# EUROPEAN PATENT SPECIFICATION

(11) **EP 2 775 986 B1**
(45) Date of publication and mention of the grant of the patent: **30.12.2020**
(21) Application number: 12784568.3
(22) Date of filing: 07.11.2012
(51) Int. Cl.: A61J 1/03, B32B 5/02, B65D 75/32, A61K 9/00, A61K 39/00

(54) **FILM PACKAGING FOR ORAL BIOLOGICS**
FOLIENVERPACKUNG FÜR ORALE BIOLOGISCHE STOFFE
EMBALLAGE EN FILM BIODÉGRADABLE POUR AGENTS BIOLOGIQUES ORAUX

(30) Priority: 07.11.2011 EP 11188099; 02.12.2011 US 201161630005 P
(43) Date of publication of application: 17.09.2014
(73) Proprietor: CEVA Santé Animale S.A., Libourne Cedex (FR)
(72) Inventor: WILKE, Jürgen, 06366 Köthen (DE); KAISER, Christian, 06046 Bernburg (DE); SCHUSTER, Peter, 06846 Dessau-Rosslau (DE)
(74) Representative: Dr. Klemens Schubert Patentanwalt
(86) International application number: PCT/EP2012/072029
(87) International publication number: WO 2013/068399

(56) References cited:
- EP-A1- 0 686 398
- US-A1- 2008 245 698

## Description

The invention relates to a sealable, molded body and the production thereof, as well as a composite film for forming the molded body and the use of the molded body for the packaging of oral biologics, in particular orally applicable vaccines. In preferred embodiments the molded body and/or the composite film are/is biologically degradable.

The problem of oral administration of agents that are biologically effective or effective in veterinary medicine (biologics, particularly vaccines) to wild animals living in the wild or in captivity, domestic animals and house pets has previously not been solved satisfactorily. Effective agents that are in liquid form or must be administered in liquid form represent a particular problem in this respect. A large and important field of application in this respect is vaccination against rabies in wild foxes. Baits have been used previously for this, and these baits contain the vaccine in a conventional blister pack enveloped by a solid vehicle substance, to which a bait or attractant or repellent is attached. These types of baits are described, for example, in DE 36 11 122 A1 or US-A 4,861,586.

The blister packs have great disadvantages, however. On the one hand, it is easy to separate them from the solid vehicle substance. Thus, the baits are no longer in a form ingested by the targeted animals, as is necessary for oral application of the vaccine. Rather, the animals separate the blister pack and then reject it.

Also, it is difficult to open the blister pack. The fox must bite into the pack in order to perforate the aluminum foil and to reach the content. The content is then taken up in the mouth cavity. In this case, small ingested quantities are generally sufficient in order to provide an effective immunization. However, the animals will only bite into the bait when it is intact and the blister pack cannot be recognized.

In contrast, this type of bait is completely unsuitable for other wild animals, since they have a different food consumption behavior. Thus, raccoons investigate nourishment very precisely and tear it into small pieces, which are then bitten into. For anatomical reasons, the form and structure of the mouth cavity does not permit taking up the bait in its entirety.

It is thus an object of the present invention to create a packaging that overcomes the disadvantages of the prior art for the oral application of biologics to animals.

### Brief description of the invention

The subject of the present invention is a container in the form of a sealable molded body, which is formed from a composite film, whereby the composite film is preferably biologically degradable, provides high barrier properties against water, oxygen, and carbon dioxide, is thermoformable and sterilizable, and has an adhesive layer for hydrophilic substances.

Disclosed is thus a sealable molded body for the packaging of oral biologics, particularly oral vaccines, formed from at least one composite film, whereby the composite film comprises two outer layers, one of the outer layers, namely the first outer layer, is formed as a sealing layer, or a barrier layer or a carrier layer, and the other outer layer, namely the second outer layer, has a rough or uneven or microstructured surface or comprises at least a nonwoven material, an adhesive material, a sticky material or a combination thereof.

The subject of the invention is a sealable molded body for the packaging of oral biologics, particularly oral vaccines, formed from at least one composite film, whereby the composite film comprises at least three layers, one of the outer layers, namely the first outer layer, being formed as a sealing layer, the other outer layer, namely the second outer layer, comprising a nonwoven material, and the at least one middle layer being a barrier layer. In that context it is preferred that the composite film is biologically degradable. It is also preferred in that context that the layers are joined with an adhesive layer. This adhesive or adhesive layer may be applied by a laminating or an extruding process, or any other process known in the art, or by a combination of the named processes.

Another subject of the present invention is a method for the production of a sealable molded body according to the invention as well as its sealing.

Finally, still another subject of the present invention is the use of a sealable molded body according to the invention for sealing pharmaceutical substances, especially oral biologics and oral vaccines.

### Brief description of the drawings

Figs. 1a and 1b show embodiment examples of the composite film according to the invention.
Figs. 2a and 2b show further embodiment examples of the composite film according to the invention.
Fig. 3 shows the arrangement of the composite film according to the invention for the structure of an embodiment of a sealable molded body according to the invention.
Figs. 4a, 4b and 4c show another embodiment of a sealable molded body according to the invention, the arrangement of the composite film according to the invention in the formation of the molded body and the molded body in the sealed state.
Fig. 5 shows the result of a Differential Scanning Calorimetry (DSC) experiment performed on composite films according to the invention.

### Detailed description of the invention

The subject of the present invention is a sealable molded body for the packaging of oral biologics, particularly oral vaccines, formed from at least one composite film, whereby the composite film comprises two outer layers, one of the outer layers, namely the first outer layer, is formed as a sealing layer, and the other outer layer, namely the second outer layer comprises at least a nonwoven material.

In order to overcome the disadvantages in the art a composite film is provided that consists of at least two layers. These two layers may be formed according to processes that are known in the art like extrusion coating or extrusion laminating and other processes or with a combination of the named or known processes. Therefore, a composite film consists of two outer layers, namely the first outer layer and the second outer layer, which may be named also as surfaces of the composite film. According to the invention these two outer layers must comprise different properties in order to solve the problem of the invention.

One outer layer, namely the first outer layer, has properties that enables the layer to undergo a sealing process. This sealing process may be accomplished by sealing two separate composite films together or by sealing a single composite film after bringing the film into a tube-like form whereby the sealing layer or surface faces itself.

The other outer layer, namely the second outer layer, has at least on the surface properties that allow other components, chemicals or substances to stick or to glue or to adhere on that surface. In order to provide these properties the other outer layer may be formed, at least on the surface, uneven, or rough, or microstructured. These surface properties may be achieved by processes known in the art, like abrasive processes, corroding processes, roughening processes, or other processes known in the art or by a combination of the known or named processes.

Furthermore, the second outer layer may also according to the invention comprise an additional material that is fixed on the surface of the other outer material. This means for example that a nonwoven material is applied on the surface of the outer layer. In other embodiments according to the present invention there may be applied an adhesive material, a sticky material or a combination thereof.

As a result the other outer layer, namely the second outer layer, shall have a property that is named in rubber industry as provided with "good grip" or in textile industry having a "good hand or handle". It is important and advantageous to provide these properties so that further material may be attached to the sealed mold in order to form a bait that may be used for the application of a biologic to a wild animal, for example.

In order to achieve the properties as described it may be advantageous that the outer layers comprise at least one sub-layer. The sub-layer has advantageously different physical and/or chemical properties in respect to the original layer. A sub-layer may be present on one side of the original layer or on both sides of the original layer.

In order to prepare such layers with different sub-layers, known processes are used. Suitable processes have been described already and comprise, for example, extrusion coating or extrusion lamination or co-extrusion processes. Processes for metallisation of films and foils are also known in the art and may be combined with the named or known processes in order to obtain a composite film according to the invention.

In an example according to the present invention a layer may be prepared from a suitable basic material like a polymer. One side of that basic layer may have sealing properties, whereby the other side of that layer may be metallised. This means that in the described outer layer the sealing function, the barrier function, and the carrier function are present in a single layer that is formed out of three sub-layers. This layer is then part of the composite film.

In a preferred embodiment the subject of the present invention is a sealable molded body for the packaging of oral biologics, particularly oral vaccines, formed from at least one composite film, whereby the composite film comprises at least three layers, one of the outer layers, namely the first outer layer, is formed as a sealing layer, the other outer layer, namely the second outer layer, comprises a nonwoven material and the at least one middle layer is a barrier layer. It is preferred that the composite film is biologically degradable. It is also preferred that adjacent layers are joined together with an adhesive layer. In that context it is especially preferred that the adhesive layer is in form of a laminating adhesive layer, or in form of an extruding adhesive layer, or in form of a combination of the before named adhesive layers.

It is also preferred according to the invention that the sealing layer, the barrier layer, the adhesive layer and/or the layer of nonwoven material are made of a native biopolymer, comprise a bio-based polymer, a petroleum-based polymer or mixtures thereof. It is especially preferred that the native biopolymer is selected from celluloses, cellulose derivatives, starches and starch derivatives; the bio-based polymer is selected from polylactides, polyhydroxybutyrates, lignin-based thermoplastics, oil-based ethoxyacrylates; and the petroleum-based polymer is selected from polyesters, polyurethanes, polyvinyl alcohols, polybutylene adipate terephthalates, polybutylene succinates, polycaprolactones and polyglycolides. In that context it is also preferred according to the invention that the polymer is metallized.

It is especially preferred according to the present invention that the adhesive is a biologically degradable polyurethane adhesive.

Preferred is also according to the invention that the barrier layer is biologically degradable and comprises a metallized cellulose derivative or a cellulose derivative containing silicon dioxide and/or aluminum oxide.

It is also preferred that the nonwoven material comprises a fleece or a web that has a fiber length of 1 to 10 cm, preferably 2 to 8 cm, and particularly preferred 4 cm.

Also preferred is that the barrier layer has a blocking effect relative to the fluids: water, air, oxygen, and carbon dioxide.

In a preferred embodiment of the present invention the sealable molded body is characterized in that the sealing layer has a thickness between 10 and 100 µm, preferably 30 to 70 µm, and particularly preferred 50 µm, the adhesive layer has a thickness between 1 and 10 µm, preferably 2 to 5 µm, and particularly preferred 3 µm, the outer layer of nonwoven material has a thickness between 0.1 and 1 mm, preferably 0.2 to 0.5 mm and particularly preferred 0.3 mm and the at least one barrier layer has a thickness between 5 and 50 µm, preferably 10 to 30 µm, and particularly preferred 20 µm.

It is also preferred that the sealable molded body according to the invention is further characterized in that the composite film contains between 1 and 5, preferably 3, and particularly preferred 2 barrier layers.

It is especially preferred that the composite film is thermoformable.

The sealable molded body according to invention is preferred in that the composite film has a tensile strength of more than 50 newtons, a tear resistance of more than 10 newtons, a bonding adhesion of more than 1.7 newtons, a permeability of a maximum 10 g/m²/24h water vapor at 38 °C and 90% relative humidity, a permeability of a maximum 3 cm³/m²/24h carbon dioxide at 23 °C and 50% relative humidity and/or a permeability of a maximum 1.5 cm³/m²/24h oxygen at 23 °C and 50% relative humidity.

It is also preferred that the physical properties of the composite film according to the invention should at least be in accordance with MIL-PRF-44073F "Performance Specification" for "Packaging of Food in Flexible Pouches".

It is also preferred that the composite film is present in the form of sheet pieces and that the molded body can be produced from two pieces of composite film. In that context it is also preferred that the composite film of the respective pieces is identical or different, at least one composite film being thermodeformable.

Another subject of the present invention is a method for producing a sealable molded body, wherein a recess is formed in a piece of the composite film by thermal forming in such a way that the outer layer of nonwoven material faces outwardly, the second piece of the composite film according to one of claims 1 to 15 is brought into contact with the first piece of composite film in such a way that the respective sealing layers face one another, and after filling the recess of the molded body, the two pieces of the composite film are sealed together by applying heat to the contact regions.

In another preferred embodiment of the present invention, the composite film that is the basis of the invention, thus the composite film from which the sealable molded body can be produced, comprises at least three layers, whereby each of the layers is joined with a laminating adhesive layer, one of the outer layers, namely the first outer layer, being formed as a sealing layer, the other outer layer, namely the second outer layer, comprising a nonwoven material, and the at least one middle layer being a barrier layer, whereby the composite film is biologically degradable.

A preferred composite film is formed in such a way that the sealing layer has a thickness between 10 and 100 µm, preferably 30 to 70 µm, and particularly preferred 50 µm, the laminating adhesive layer has a thickness between 1 and 10 µm, preferably 2 to 5 µm, and particularly preferred 3 µm, the outer layer of nonwoven material has a thickness between 0.1 and 1 mm, preferably 0.2 to 0.5 mm and particularly preferred 0.3 mm, and the at least one barrier layer has a thickness between 5 and 50 µm, preferably 10 to 30 µm, and particularly preferred 20 µm.

In addition, a preferred composite film contains 1 to 5, preferably 3, and particularly preferred 2 barrier layers.

According to the invention, the composite film is formed in such a way that one outer side of the composite film is made sealable. These types of sealable formations are known to the person skilled in the art. This side of the composite film is directly in contact with the goods to be packaged. In this case, it is provided that the material of the side that is directly in contact with the goods to be packaged is selected in such a way that no interaction occurs with the goods to be packaged. It therefore must be excluded that the packaged goods will be damaged by the packaging or their activity will be adversely affected.

In connection with the present invention, however, the term "sealable" is also understood as any configuration of the outside of the composite film that is suitable to be joined with the outside of another composite film. This joining can be effected by adhesion, contact adhesion or bonding of the respective outer sides with one another. In this case corresponding glues or adhesives or adhesion agents known in the state of the art can be used.

In addition, the composite film has a barrier layer. This barrier layer ensures that media from the environment, such as water, water vapor, air, oxygen, carbon dioxide, and other substances present in the atmosphere do not enter inside the packaging formed from the composite film to reach the packaged goods and damage the packaged goods. On the other hand, the barrier layer ensures that components of the packaged goods, particularly fluids and/or liquids like water, cannot leak out from inside the blister.

According to the invention, it is further essential that the second outer side of the composite film comprises a nonwoven material or a nonwoven material is applied onto the outer side. This nonwoven material represents the surface of the formed packaging that does not come in contact with the goods to be packaged.

The entire composite film is biologically degradable according to the invention. This property of biological degradability is defined by the standards EN 13432, ASTM D.6400-99 UNI 11183:2006 or AS 4736-2006. The biological degradability is achieved in that the sealing layer, the barrier layer, the adhesive layer(s) and the layer of nonwoven material comprise a native biopolymer, a bio-based polymer, a petroleum-based polymer or mixtures thereof. Here, it is preferred that the adhesive is a biologically degradable adhesive, for example, a polyurethane adhesive.

In this case, the native biopolymer is selected from celluloses, cellulose derivatives, starches and starch derivatives; the bio-based polymer is selected from polylactides, polyhydroxybutyrates, lignin-based thermoplastics, oil-based ethoxyacrylates; and the petroleum-based polymer is selected from polyesters, polyurethanes, polyvinyl alcohols, polybutylene adipate terephthalates, polybutylene succinates, polycaprolactones and polyglycolides. In this context is should be understood that paper or paper fibers may also be selected as native biopolymers. Other suitable biologically degradable polymers are known in the prior art, such as those that are described, for example, in DE 196 30 235 A1, DE 198 11 773 A1 and DE 198 11 226 A1.

The nonwoven material present on the outer side of the composite film or forming the outer side of the composite film is also biologically degradable. Appropriate nonwoven materials, for example, are known from DE 44 09 465 A1. The nonwoven material contains at least one degradable polymer/copolymer fiber, or a polymer/copolymer fiber that is degradable by means of contained starches and/or added fermented starches, selected from natural polymers or natural polymers modified with synthetic polymers and/or synthetic polymer fibers, which have been modified by biological starches, e.g., of potatoes, beets, sugar cane, corn, wheat and the like, in particular those of polyolefins and/or polyesters/copolyesters, which can be obtained, e.g., from starches, fermented starch products or plants modified by strains of bacteria, such as potatoes, rapeseed, etc., also in combination with several, possibly different varieties of biologically degradable polymer fibers and/or natural fibers or copolyamides and/or biological fibers, such as jute, hemp, flax or the like and/or degradable aggregates and/or degradable films. The fleece or the nonwoven material can be configured as hydrophobic or hydrophilic, depending on the respective field of application.

The nonwoven fibers can be produced from a film, e.g., by fibrillating, but may also comprise spun fibers, which, as fleece or nonwoven material, are mechanically reinforced, e.g., by needling or water-jet reinforcement (spun lace method), or are thermally reinforced or are reinforced by binders, or are reinforced by a mixture of all of these possibilities. In this case, the films and also the spun fibers can be stretched in advance in one or several dimensions. The fleece or the nonwoven material can then be applied onto a layer, for example, by means of an adhesive. In this case, it is preferred that the nonwoven material comprises a fleece that has a fiber length of 1 to 10 cm, preferably 2 to 8 cm, and particularly preferred 4 cm.

In order to improve the properties of the composite film, it is provided that at least one of the layers of the composite film is formed as a barrier layer. In addition to the property of biological degradability, which is inherent due to the polymer itself, aggregates are added to the barrier layer, or metal and/or oxide materials are applied onto the barrier layer in order to provide or to improve the barrier properties. A barrier layer formed from polyvinyl alcohol is known, for example, from DE 196 32 799 A1. Appropriate barrier layers containing metal or oxide materials are known from DE 43 28 767 A1. Particularly suitable in the case of metals are silver, aluminum, iron and the like, while in the case of oxide compounds, the following are particularly suitable: aluminum oxide, cerium oxide, hafnium oxide, magnesium oxide, silicon dioxide, silicon monoxide, tantalum oxide, titanium dioxide, titanium (III) oxide, titanium monoxide, yttrium oxide, zirconium oxide, zirconium monoxide and the like, or mixtures thereof. It is particularly preferred that the barrier layer is biologically degradable and comprises a cellulose derivative, which is metallized or contains silicon dioxide and/or aluminum oxide.

In order to assure the required use properties of the composite film and the sealable or sealed molded body produced therefrom, it is advantageous that the composite film has a tensile strength of more than 50 newtons, a tear resistance of more than 10 newtons, a bonding adhesion of more than 1.7 newtons, a permeability of a maximum 10 g/m²/24h water vapor at 38 °C and 90% relative humidity, a permeability of a maximum 3 cm³/m²/24h carbon dioxide at 23 °C and 50% relative humidity and/or a permeability of a maximum 1.5 cm³/m²/24h oxygen at 23 °C and 50% relative humidity.

It is important to emphasize in this connection that the barrier properties of the composite film should be effective in both directions. It is important to protect the goods contained in the molded body from environmental influences, thus from influences that act from the outside. However, it must also be ensured that the goods contained in the molded body do not leak out of the molded body.

The described composite film is especially suitable for forming a sealable molded body, which is then provided in sealed form for the oral application of biologics.

Therefore, a subject of the present invention is a sealable molded body, which is formed from two sheet pieces of the composite film described herein. In this case, it is provided according to the invention that the composite film of the individual pieces is identically or differently constructed. This means that the sequence of layers or the number of barrier layers can be configured differently in one composite film than in the other composite film. In any case, at least one composite film must be configured thermoformable in order to introduce the active substance therein. It may be provided according to the invention, however, to thermally form both pieces of one composite film, whereby one composite film may be identical or different than the other composite film also in this case. It will then be advantageous to thermally form both pieces if larger quantities of active substances are to be introduced.

The production of a sealable molded body according to the invention results in the fact that a recess is formed in one piece of the composite film by thermal forming in such a way that the outer layer of nonwoven material faces the outside, the second piece of the same composite film or a modified composite film is brought into contact with the first piece of the composite film, that the respective sealing layers face each other and after filling the molded body, the two pieces of the composite film are sealed together by application of heat to the contact regions. It is particularly preferred according to the invention that each of the two pieces of the composite film forms a recess by thermoforming.

The production of a sealable molded body according to the invention, however, can also result in the circumstance that the composite film from which the molded body is formed does not have a sealing layer that can be thermally sealed. In this case, the molded body is first formed by thermoforming, just as described previously, and after this is provided with an adhesive, glue or adhesion layer and only then, optionally after filling the molded body with the goods to be packaged, is closed (sealed), by introducing another composite film. This method variant may be advantageous if the application of heat might lead to an adverse effect on the goods being packaged in the case of the usual sealing layers. The use of an adhesive, glue or adhesion layer may also be limited in this case to the region of the actual contact places.

It is also provided according to the invention to form the molded body in such a way that an excess of material of one of the pieces remains in the region of the sealing seam. For this purpose, for example, pieces with different widths are sealed together. The excess of material projecting from the sealing seam can then be printed on by means of suitable methods. The material excess projecting from the sealing seam is preferably dimensioned in such a way that, along with the printing that may be present, it also projects out from the entire bait, i.e., the molded body enveloped with the bait material and is visible.

According to the present invention it is also possible to form the molded body in order to obtain a sachet or a pillow pack or a stick pack. This means that any package form that is known in the art is suitable and in within the scope of the present invention.

It is also possible and within the scope of the present invention to form a molded body in form of a sachet or in form of a pillow pack. This means that two composite films are sealed together in order to form a rectangular or square mold that has sealing seam on the four sides of the sachet or pillow.

It is also envisaged according to the invention that the composite film may be molded in form of so-called stick packs as they are currently used for packaging sugar (sugar stick or sugar stick pack). In that case only one composite film is needed and has to be folded or bend along the running direction of the film in order to provide a tube-like mold. This tube-like mold may then be separated into single molds that can be filled with the biologic substance or the like and afterwards being sealed and cut into single dosage forms for further processing.

The sealable or sealed molded body according to the invention is suitable in a particular way for the oral application of biologics.

Agents that are biologically active or effective in veterinary medicine are understood here as biologics, these being produced in classical and/or biotechnological ways. Included here, in particular, are human pharmaceuticals, veterinary drugs, contraceptives and vaccines in the form of killed vaccines, living vaccines or attenuated living vaccines, produced with classical or biotechnological methods. The active substances prepared correspondingly, in addition to the actual active substances, can also contain additives, excipients and adjuvants that are physiologically tolerable, thus do not act in themselves in a damaging way.

Under the term "oral" or "oral application", in the sense of the invention, is understood the administration of substances by passing a mouth of a human or an animal (per os). Therefore, the substance can be released and/or resorbed in the mouth cavity. Oral biologics in the sense of the invention, however, also comprise preparations that are only released and/or resorbed in the stomach or in the intestinal tract.

The molded body itself is produced from biologically degradable materials. Therefore, the introduction of molded bodies filled with active substances into the environment, even in relatively large quantities, is ecologically unobjectionable. Testing for biological degradability can be carried out according to the criteria of relevant standards, such as EN 13432, ASTM D.6400-99 UNI 11183:2006 or AS 4736-2006.

In addition, the molded bodies can be sterilized. Depending on the active substance to be applied, the preparation and/or the entire filled and sealed molded body must be subjected to a sterilization. The sterilization can be conducted by thermal or chemical methods or by energy-rich irradiation. The material of the composite film must be stable relative to the sterilization.

Due to the barrier layer, the molded body and/or the composite film are/is nearly impermeable to substances acting from the environment, such as water, water vapor, oxygen, carbon dioxide and other substances contained in the atmosphere. The biologics present in the molded body are frequently very sensitive and can be damaged and lose their effectiveness due to the action of the named substances. Therefore, the biologics must be protected from these environmental influences.

The composite film is formed in such a way that the polymer layer that comes into contact with the biologics does not interact with them and, in particular, does not exercise a damaging effect.

The nonwoven material provided on the outer side of the molded body finally fulfills different tasks. The nonwoven material can be configured hydrophobic, hydrophilic, lipophobic or lipophilic, depending on the selection of the initial materials used. Further, the nonwoven material represents an enlarged effective surface for the molded body. If the molded body is opened or bursts open due to the action of the targeted animal, then the preparation contained therein in liquid or viscous form does not completely run out, but is absorbed to a considerable extent by the nonwoven material. This means that, in addition, the preparation remains in the region of contact with the targeted animal and thus the probability of oral ingestion of the biologic by the targeted animal is increased. This effect can be reinforced by the appropriate galenic preparation of the biologic, such as increasing the viscosity in the case of liquids.

The nonwoven material, however, can also be suitable for improving the adhesion of other materials to the surface by an appropriate configuration of the surface properties. Thus, the molded body can be introduced, for example, into bait, whereby the bait material is a fat, for example. The surface properties can be correspondingly adjusted or selected also for other bait materials such as proteins, carbohydrates and the like, in order to improve the adhesion. In this way, adhesion of the bait material to the surface of the molded body is reinforced and separation of the two components of the bait is made difficult.

Finally, the use of a sealable molded body according to the present invention as described herein sealing pharmaceutical substances, especially oral biologics and oral vaccines is another subject of the present invention.

The invention will now be explained in more detail on the basis of the figures and the embodiment examples introduced therein.

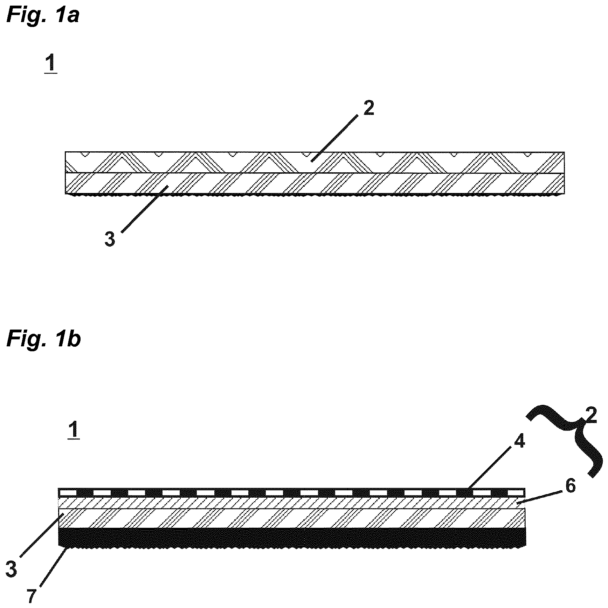

Figures 1a and 1b show embodiment examples of composite film 1 according to the invention. In Figure 1a composite film 1 is shown comprising two outer layers 2 and 3. A composite film 1 comprising two outer layers is the simplest form of a composite film. Composite films in general comprise at least two layers and these two layers are naturally outer layers.

According to the invention, the outer layers 2 must comprise certain properties. One outer layer 2, namely the first outer layer, must have at least sealing capabilities. This means that this layer 2 may be sealed with another composite film having an outer layer with sealing capabilities or that the outer layer 2 with sealing capabilities may be sealed with the same outer layer 2 after changing the geometrical arrangement of that outer layer 2 by forming a tube, for example. Beside the sealing capabilities of outer layer 2, outer layer 2 two may also be formed as a barrier layer or a carrier layer. It is also possible and within the scope of the present invention that these capabilities are inherently combined in one single layer, which then forms outer layer 2.

According to the invention, the other outer layer 3, namely the second outer layer, must also comprise certain properties. As shown in Figure 1a, the other outer 3 layer has a rough or uneven or microstructured surface. This kind of a surface modification is essential in order to adhere further material that may be part of a bait. As described herein already, the sealed molded body of the invention is part of a bait that has to be formed around the molded body. This bait is then used to administer the biologics contained in the sealed molded body to an animal. Composite films known in the art do not have any uneven or rough or microstructured surface at all.

In Figure 1b a further embodiment of the present invention is shown. Outer layer 2, the first outer layer, now comprises two sub-layers 4 and 6. Sub-layer 4 is a sealing layer, whereas sub-layer 6 is a barrier or carrier layer. It is possible to form such outer layers 2, comprising two sub-layers 4 and 6 by a co-extrusion process, for example. This means that outer layer 2 now also shows the sealing properties that are entered into outer layer 2 by means of a sealing sub-layer 4. Outer layer 3 comprises a nonwoven material 7. This is also shown in Figure 1b.

In order to achieve the adhering properties of the surface of outer layer 3 it is according to the invention also possible to modify the outer layer 3. In that case the outer layer 3 comprises at least a nonwoven material 7, an adhesive material (not shown), a sticky material (not shown) or a combination thereof.

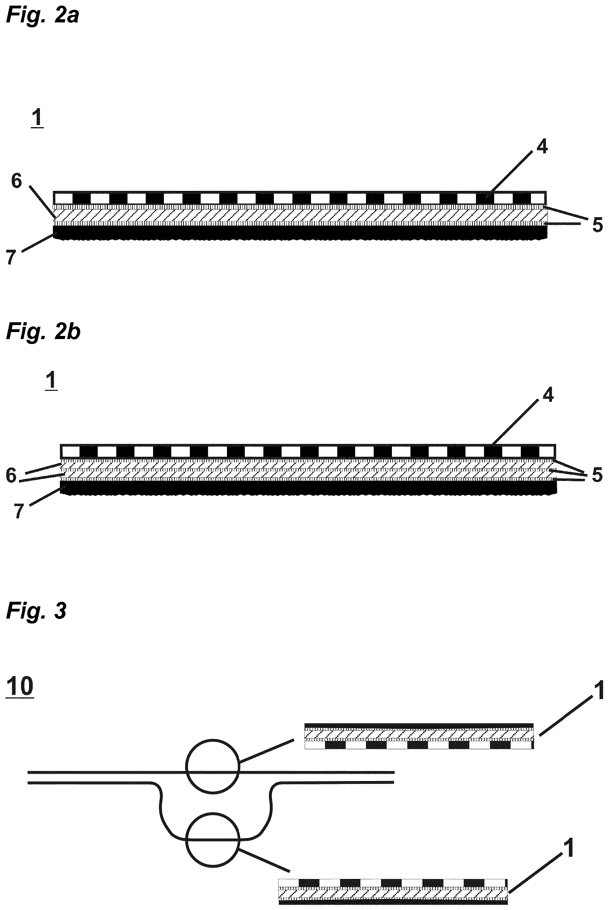

Figures 2a and 2b show further preferred embodiment examples of composite film 1 according to the invention. Composite film 1 according to the invention, which is shown in Figure 2a, is composed of three layers, each of which is joined to the adjacent layer by a laminating adhesive layer 5. One of the two outer layers is formed as a sealing layer 4. The second outer layer 7 is formed of nonwoven material. A barrier layer 6 is found between these layers.

An example of embodiment of composite film 1 according to the invention, which has more than one barrier layer 6, is shown in Figure 2b. Shown is a composite film 1 having two barrier layers 6, which are joined together by laminating adhesive layers 5. One of the barrier layers is joined to sealing layer 4 by a laminating adhesive layer 5, and one of barrier layers 6 is joined to the outer layer 7, which comprises nonwoven material, by a laminating adhesive layer 5.

Figure 3 shows an example of embodiment of a sealable molded body 10 according to the invention. Regions of these films, which are also shown enlarged, are characterized in each case by a circle in this figure. Molded body 10 is formed by two sheet pieces of composite film 1 according to the invention. The part of molded body 10, which is shown in the lower region of the figure and which is formed by a piece of film, has a depression or recess, as can be produced by thermoforming composite film 1. A second piece of composite film 1 according to the invention is shown in the upper region of the figure. The two pieces of film of composite film 1 according to the invention are aligned relative to one another so that each of sealing layers 2 are facing each other. In the enlargements, the schematic structure of composite film 1 is shown, as it is shown also in Figure 2a and within the scope of the description of this figure. As an example, an embodiment of composite film having one barrier layer 4 is shown. Molded body 10, however, may also be formed by any other embodiment of composite film 1 according to the invention.

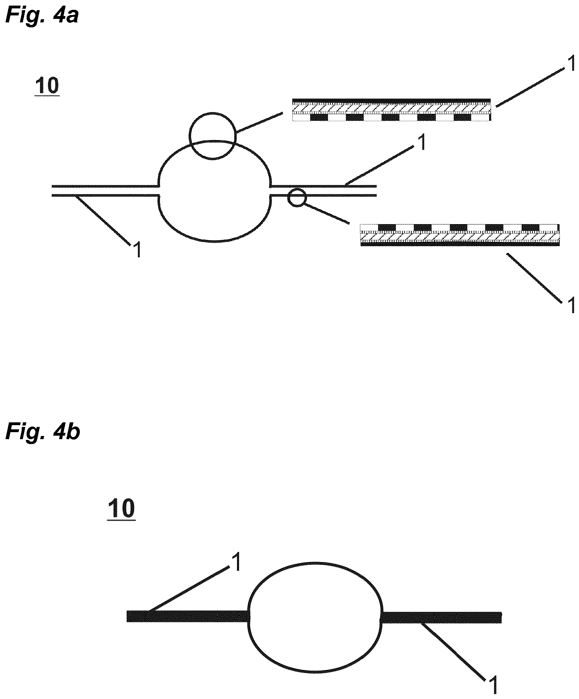

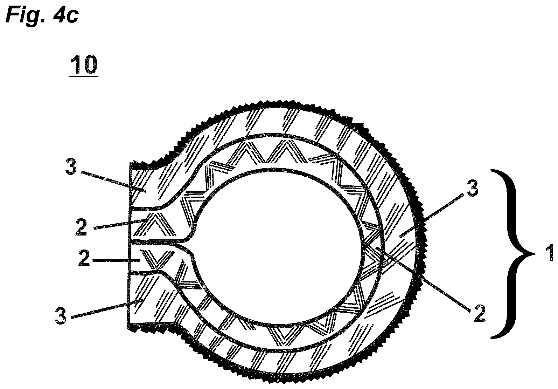

Another embodiment of sealable molded body 10 according to the invention is shown in Figures 4a, 4b and 4c. As in the case of the example of embodiment of Figure 3, this molded body is also formed by two pieces of film of composite film 1 according to the invention. In Figures 4a and 4b, however, an embodiment is shown, in which both pieces of film have been formed prior to sealing. A depression or recess is formed in both pieces of film. Shown is an embodiment in which both parts of sealable molded body 10 are identical. The exemplary structure possessed by the pieces of film of composite film 1 according to the invention is shown in the enlarged excerpts of Figure 4a.

Figure 4a shows sealable molded body 10, in which the pieces of composite film 1 according to the invention have not as yet been brought into contact with one another. The pieces of composite film 1 are already aligned relative to one another in such a way that the molded body can be sealed.

It is shown in Figure 4b how both pieces of film of composite film 1 according to the invention have been brought into contact with one another in order to form the sealable molded body. In practice, when the pieces of film are arranged in this way, the molded body will already have been filled and subsequently will be sealed.

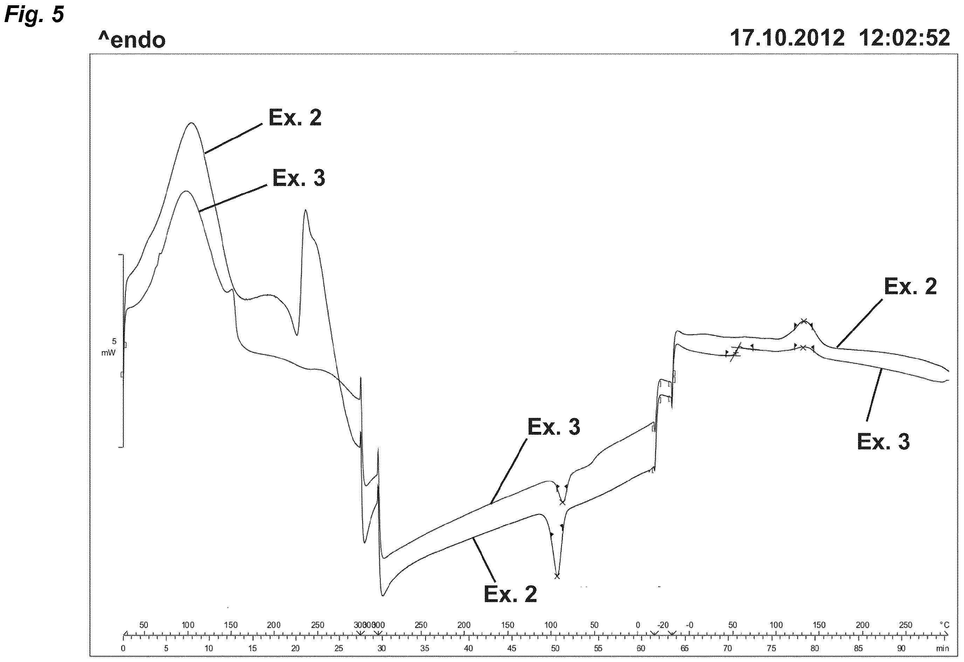

Finally, in Figure 4c it is shown, that by rolling in a composite film 1 also a mold or a sealable molded body 10 may be formed. In this embodiment outer layer 2 faces itself and can be sealed to form a seam-like structure. The resulting tube-like mold can then be separated by additional sealing along the running direction of the tube-like structure (sugar sticks or stick packs). It is also apparent from Figure 4c that the other outer layer 3 now faces outwards and can be used for further adhering of bait material. In order to achieve this a nonwoven material 7 (not shown) may be comprised in the outer layer 3. Figure 5 shows the result of a Differential Scanning Calorimetry (DSC) experiment performed on composite films according to the invention as laid out in Examples 2 and 3 as described herein.

The following examples explain the present invention in more detail.

### Example 1

### General layout of a first embodiment

A molded body according to the invention is formed as follows:
Molded bodies made of composite films, which are sealed with one another, are biologically degradable and thermoformable, provide the following layer structure, from inside to outside:
- Sealing layer of biologically degradable polyester
- Biologically degradable two-component adhesive having reactive N-CO group
- Barrier layer of biologically degradable, metallized cellulose layer
- Biologically degradable two-component adhesive having reactive N-CO group
- Attractive-material adhesion layer of biologically degradable nonwoven material made of viscose fibers

### Example 2

### General layout of a second embodiment

Composite film providing the following layer structure from inside to outside:
- Sealing layer of biologically degradable polyester attached to barrier layer by extrusion laminating process
- Barrier layer of biologically degradable, metallized PVdC layer
- Sealing layer of biologically degradable polyester attached to other side of barrier layer by extrusion laminating process
- Attractive-material adhesion layer of biologically degradable nonwoven material (mixture of viscose and cellulose (1:1 /w:w) fibers)

### Example 3

### General layout of a third embodiment

Composite film providing the following layer structure from inside to outside:
- Sealing layer of biologically degradable polyester attached to barrier layer by extrusion coating process
- Barrier layer of biologically degradable, metallized polylactide blend layer (Coex foil)
- Sealing layer of biologically degradable polyester attached to other side of barrier layer by extrusion laminating process
- Attractive-material adhesion layer of biologically degradable nonwoven material made of viscose fibers

### Example 4

### DSC experiments

Figure 5 shows DSC experiments performed with composite films formed according to Examples 2 and 3.

Experiments were performed with a DSC 204 F1 - Differential Scanning Calorimeter (Manufacturer: NETZSCH Gerätebau GmbH, Germany) at a heating rate of 10 K/min in a range from 0 to 300 °C under N₂. Two heating cycles were performed, interrupted by one cooling cycle in the temperature range from 300 to -20 °C.

As shown in Figure 5, both samples, marked with Ex. 2 and Ex. 3, show a peak in the first heating cycle at about 100 °C. This peak is believed to be associated with the loss of humidity present in the samples. During the cooling cycle both samples show a crystallization peak at nearly the same temperature. In the second heating cycle both samples also show a crystallite melting peak at nearly the same temperature.

Surprisingly it was found that even if the structure and or the chemical composition of the composite films are extremely different, the thermal behavior of the composite films according to the invention are quite similar after the composite has been reconditioned during a first heating cycle.

### List of reference numbers

- 1: Composite film
- 2: 1^{st} outer layer
- 3: 2^{nd} outer layer
- 4: Sealing layer
- 5: Laminating adhesive layer
- 6: Barrier layer / carrier layer
- 7: Layer of nonwoven material
- 10: Sealable molded body

## Claims

1. A sealable molded body for the packaging of oral biologics, particularly oral vaccines, formed from at least one composite film,
whereby the composite film comprises at least three layers,
namely two outer layers and at least one middle layer,
wherein the first outer layer is formed as a sealing layer,
the second outer layer comprises at least a nonwoven material or a nonwoven material is applied onto the second outer layer,
the nonwoven material being biodegradable,
and the at least one middle layer is a barrier layer,
and wherein the second outer layer has at least on the surface properties that allow other components, chemicals or substances to stick or to glue or to adhere on that surface.

2. The sealable molded body according to claim 1, wherein the composite film is biologically degradable.

3. The sealable molded body according to claims 1 or 2, wherein adjacent layers are joined together with an adhesive layer.

4. The sealable molded body according to claim 3, wherein the adhesive layer is in form of a laminating adhesive layer, or in form of an extruding adhesive layer, or in form of a combination of the before named adhesive layers.

5. The sealable molded body according to one of the preceding claims, further **characterized in that** the sealing layer, the barrier layer, the adhesive layer, the nonwoven material, the adhesive material, and/or the sticky material are made of a native biopolymer, comprising a bio-based polymer, a petroleum-based polymer or mixtures thereof.

6. The sealable molded body according to claim 5, further **characterized in that** the native biopolymer is selected from celluloses, cellulose derivatives, starches and starch derivatives; the bio-based polymer is selected from polylactides, polyhydroxybutyrates, lignin-based thermoplastics, oil-based ethoxyacrylates; and the petroleum-based polymer is selected from polyesters, polyurethanes, polyvinyl alcohols, polybutylene adipate terephthalates, polybutylene succinates, polycaprolactones and polyglycolides.

7. The sealable molded body according to claim 5 or 6, further **characterized in that** the polymer is metallized.

8. The sealable molded body according to one of the claims 3 to 7, further **characterized in that** the adhesive is a biologically degradable polyurethane adhesive.

9. The sealable molded body according to one of the preceding claims, further **characterized in that** the barrier layer is biologically degradable and comprises a metallized cellulose derivative or a cellulose derivative containing silicon dioxide and/or aluminum oxide.

10. The sealable molded body according to one of the preceding claims, further **characterized in that** the nonwoven material comprises a fleece that has an average fiber length of 1 to 10 cm, preferably 2 to 8 cm, and particularly preferred 4 cm.

11. The sealable molded body according to one of the preceding claims, further **characterized in that** the barrier layer has a blocking effect relative to the fluids, namely water, air, oxygen, and carbon dioxide.

12. The sealable molded body according to one of the preceding claims, further **characterized in that**
the sealing layer has an average thickness between 10 and 100 µm, preferably 30 to 70 µm, and particularly preferred 50 µm,
the adhesive layer has a thickness between 1 and 10 µm, preferably 2 to 5 µm, and particularly preferred 3 µm,
the outer layer of nonwoven material has a thickness between 0.1 and 1 mm, preferably 0.2 to 0.5 mm and particularly preferred 0.3 mm and the at least one barrier layer has a thickness between 5 and 50 µm, preferably 10 to 30 µm, and particularly preferred 20 µm.

13. The sealable molded body according to one of the preceding claims, further **characterized in that** the composite film contains between 1 and 5, preferably 3, and particularly preferred 2 barrier layers.

14. The sealable molded body according to one of the preceding claims, further **characterized in that** the composite film is thermoformable.

15. The sealable molded body according to one of the preceding claims, further **characterized in that** the composite film has a tensile strength of more than 50 newtons, a tear resistance of more than 10 newtons, a bonding adhesion of more than 1.7 newtons, a permeability of a maximum 10 g/m²/24h water vapor at 38 °C and 90% relative humidity, a permeability of a maximum 3 cm³/m²/24h carbon dioxide at 23 °C and 50% relative humidity and/or a permeability of a maximum 1.5 cm³/m²/24h oxygen at 23 °C and 50% relative humidity.

16. The sealable molded body according to one of the preceding claims, further **characterized in that** the composite film is present in the form of sheet pieces and that the molded body can be produced from two pieces of composite film.

17. The sealable molded body according to claim 16, further **characterized in that** the composite film of the respective pieces is identical or different, at least one composite film being thermodeformable.

18. A method for producing a sealable molded body according to claim 16, wherein
a recess is formed in a piece of the composite film by thermal forming in such a way that the outer layer of nonwoven material faces outwardly,
the second piece of the composite film according to one of claims 1 to 15 is brought into contact with the first piece of composite film in such a way that the respective sealing layers face one another, and
after filling the recess of the molded body, the two pieces of the composite film are sealed together by applying heat to the contact regions.

19. Use of a sealable molded body according to any of the claims 1 to 17 for sealing pharmaceutical substances, especially oral biologics and oral vaccines.

## Patentansprüche

1. Versiegelbarer Formkörper zur Verpackung für orale Biologika, insbesondere orale Impfstoffe, gebildet aus mindestens einem Folienverbund,
wobei der Folienverbund mindestens drei Schichten umfasst,
nämlich zwei äußere Schichten und mindestens eine mittlere Schicht, worin die erste äußere Schicht als Siegelschicht ausgebildet ist,
die zweite äußere Schicht mindestens einen Vliesstoff umfasst oder ein Vliesstoff auf der zweiten äußeren Schicht aufgebracht ist,
der Vliesstoff biologisch abbaubar ist,
und die mindestens eine mittlere Schicht eine Barriereschicht ist,
und worin die zweite äußere Schicht mindestens auf der Oberfläche Eigenschaften besitzt, die es anderen Komponenten, Chemikalien oder Substanzen ermöglichen, an dieser Oberfläche anzuhaften, festzukleben oder hängenzubleiben.

2. Versiegelbarer Formkörper gemäß Anspruch 1, worin der Folienverbund biologisch abbaubar ist.

3. Versiegelbarer Formkörper gemäß Anspruch 1 oder 2, worin benachbarte Schichten mit einer Kleberschicht miteinander verbunden sind.

4. Versiegelbarer Formkörper gemäß Anspruch 3, worin die Kleberschicht in Form einer Kaschierkleber-Schicht oder in Form einer Extrusionskleber-Schicht oder einer Kombination der vorgenannten Kleberschichten vorliegt.

5. Versiegelbarer Formkörper gemäß einem der vorangehenden Ansprüche, weiterhin **dadurch gekennzeichnet, dass** die Siegelschicht, die Barriereschicht, die Kleberschicht, der Vliesstoff, das Klebemittel und/oder das Haftmittel aus einem natürlichen Biopolymer hergestellt sind, umfassend ein biobasiertes Polymer, ein erdölbasiertes Polymer oder deren Mischungen.

6. Versiegelbarer Formkörper gemäß Anspruch 5, weiterhin **dadurch gekennzeichnet, dass** das natürliche Biopolymer ausgewählt ist aus Zellulosen, Zellulosederivaten, Stärken und Stärkederivaten; das biobasierte Polymer ausgewählt ist aus Polylaktiden, Polyhydroxybutyraten, Lignin-basierten Thermoplasten, ölbasierten Ethoxyacrylaten; und das erdölbasierte Polymer ausgewählt ist aus Polyestern, Polyurethanen, Polyvinylalkoholen, Polybutylenadipatterephthalaten, Polybutylensuccinaten, Polycaprolactonen und Polyglykoliden.

7. Versiegelbarer Formkörper gemäß Anspruch 5 oder 6, weiterhin **dadurch gekennzeichnet, dass** das Polymer metallisiert ist.

8. Versiegelbarer Formkörper gemäß einem der Ansprüche 3 bis 7, weiterhin **dadurch gekennzeichnet, dass** der Klebstoff ein biologisch abbaubarer Polyurethan-Klebstoff ist.

9. Versiegelbarer Formkörper gemäß einem der vorangehenden Ansprüche, weiterhin **dadurch gekennzeichnet, dass** die Barriereschicht biologisch abbaubar ist und ein metallisiertes Zellulosederivat, oder ein Zellulosederivat umfasst, das Siliziumdioxid und/oder Aluminiumoxid enthält.

10. Versiegelbarer Formkörper gemäß einem der vorangehenden Ansprüche, weiterhin **dadurch gekennzeichnet, dass** der Vliesstoff ein Vlies umfasst, das eine mittlere Faserlänge von 1 bis 10 cm, bevorzugt 2 bis 8 cm und insbesondere 4 cm hat.

11. Versiegelbarer Formkörper gemäß einem der vorangehenden Ansprüche, weiterhin **dadurch gekennzeichnet, dass** die Barriereschicht eine Sperrwirkung in Bezug auf Fluide, nämlich Wasser, Luft, Sauerstoff und Kohlendioxid hat.

12. Versiegelbarer Formkörper gemäß einem der vorangehenden Ansprüche, weiterhin **dadurch gekennzeichnet, dass** die Siegelschicht eine mittlere Dicke zwischen 10 und 100 µm, bevorzugt 30 bis 70 µm, und insbesondere bevorzugt 50 µm hat, die Kleberschicht eine Dicke zwischen 1 und 10 µm, bevorzugt 2 bis 5 µm, und insbesondere bevorzugt 3 µm hat,
die äußere Schicht aus Vliesstoff eine Dicke zwischen 0,1 und 1 mm, bevorzugt 0,2 bis 0,5 mm und insbesondere bevorzugt 0,3 mm hat, und die mindestens eine Barriereschicht eine Dicke zwischen 5 und 50 µm, bevorzugt 10 bis 30 µm und insbesondere bevorzugt 20 µm hat.

13. Versiegelbarer Formkörper gemäß einem der vorangehenden Ansprüche, weiterhin **dadurch gekennzeichnet, dass** der Folienverbund zwischen 1 und 5, bevorzugt 3 und insbesondere bevorzugt 2 Barriereschichten enthält.

14. Versiegelbarer Formkörper gemäß einem der vorangehenden Ansprüche, weiterhin **dadurch gekennzeichnet, dass** der Folienverbund thermoformbar ist.

15. Versiegelbarer Formkörper gemäß einem der vorangehenden Ansprüche, weiterhin **dadurch gekennzeichnet, dass** der Folienverbund eine Zugfestigkeit von mehr als 50 Newton, eine Weiterreißfähigkeit von mehr als 10 Newton, eine Verbundhaftung von mehr als 1,7 Newton, eine Permeabilität von maximal 10 g/m²/24h Wasserdampf bei 38 °C und 90% relativer Luftfeuchtigkeit, eine maximale Permeabilität von 3 cm³/m²/24h Kohlendioxid bei 23 °C und 50% relativer Luftfeuchtigkeit und/oder eine maximale Permeabilität von 1,5 cm³/m²/24h Sauerstoff bei 23 °C und 50% relativer Luftfeuchtigkeit besitzt.

16. Versiegelbarer Formkörper gemäß einem der vorangehenden Ansprüche, weiterhin **dadurch gekennzeichnet, dass** der Folienverbund in Form von Bahnstücken vorliegt, und dass der Formkörper aus zwei Stücken Folienverbund hergestellt werden kann.

17. Versiegelbarer Formkörper gemäß Anspruch 16, weiterhin **dadurch gekennzeichnet, dass** der Folienverbund der jeweiligen Stücke identisch oder verschieden ist, und mindestens ein Folienverbund thermoverformbar ist.

18. Verfahren zur Herstellung eines versiegelbaren Formkörpers gemäß Anspruch 16, worin
eine Aufnahme in einem Stück des Folienverbunds durch thermische Verformung derart geformt wird, dass die äußere Schicht aus Vliesstoff nach außen weist,
das zweite Stück des Folienverbunds gemäß einem der Ansprüche 1 bis 15 derart mit dem ersten Stück des Folienverbunds in Kontakt gebracht wird, dass die jeweiligen Siegelschichten aufeinander zuweisen,
nach dem Befüllen der Aufnahme des Formkörpers, die zwei Stücke des Folienverbunds durch Wärmeanwendung an den Kontaktbereichen miteinander versiegelt werden.

19. Verwendung eines versiegelbaren Formkörpers, gemäß einem der Ansprüche 1 bis 17 zum Einschließen von pharmazeutischen Substanzen, insbesondere oralen Biologika und oralen Impfstoffen.

## Revendications

1. Un corps moulé scellable pour l'emballage de produits biologiques oraux, en particulier de vaccins oraux, formé d'au moins un film composite,
le film composite comprenant au moins trois couches,
à savoir deux couches extérieures et au moins une couche médiane,
la première couche extérieure étant formée en tant que couche d'étanchéité, la deuxième couche extérieure comprenant au moins un matériau non tissé, ou un matériau non tissé étant appliqué sur la deuxième couche extérieure, le matériau non tissé étant biodégradable,
et ladite au moins une couche médiane est une couche barrière,
et la deuxième couche externe ayant au moins sur la surface des propriétés qui permettent à d'autres composants, produits chimiques ou substances de tenir ou de coller ou d'adhérer sur cette surface.

2. Le corps moulé scellable selon la revendication 1, dans lequel le film composite est biologiquement dégradable.

3. Le corps moulé scellable selon la revendication 1 ou la revendication 2, dans lequel des couches adjacentes sont réunies au moyen d'une couche adhésive.

4. Le corps moulé scellable selon la revendication 3, dans lequel la couche adhésive est sous la forme d'une couche adhésive de stratification, ou sous la forme d'une couche adhésive d'extrusion, ou sous la forme d'une combinaison des couches adhésives mentionnées ci-avant.

5. Le corps moulé scellable selon l'une des revendications précédentes, **caractérisé en outre en ce que** la couche d'étanchéité, la couche barrière, la couche adhésive, le matériau non tissé, le matériau adhésif et / ou le matériau adhésif sont constitués d'un biopolymère natif, comprenant un polymère biosourcé, un polymère à base de pétrole ou des mélanges de ceux-ci.

6. Le corps moulé scellable selon la revendication 5, **caractérisé en outre en ce que** le biopolymère natif est choisi parmi les celluloses, les dérivés cellulosiques, les amidons et les dérivés d'amidon ; le polymère biosourcé est choisi parmi les polylactides, les polynydroxybutyrates, les thermoplastiques à base de lignine, les éthoxyacrylates à base d'huile ; et le polymère à base de pétrole est choisi parmi les polyesters, les polyuréthanes, les alcools polyvinyliques, les polybutylène adipate téréphtalates, les polybutylène succinates, les polycaprolactones et les polyglycolides.

7. Le corps moulé scellable selon la revendication 5 ou la revendication 6, **caractérisé en outre en ce que** le polymère est métallisé.

8. Le corps moulé scellable selon l'une des revendications 3 à 7, **caractérisé en outre en ce que** l'adhésif est un adhésif polyuréthane biologiquement dégradable.

9. Le corps moulé scellable selon l'une des revendications précédentes, **caractérisé en outre en ce que** la couche barrière est biologiquement dégradable et comprend un dérivé cellulosique métallisé ou un dérivé cellulosique contenant du dioxyde de silicium et / ou de l'oxyde d'aluminium.

10. Le corps moulé scellable selon l'une des revendications précédentes, **caractérisé en outre en ce que** le matériau non tissé comprend un non-tissé qui a une longueur moyenne de fibre de 1 à 10 cm, de préférence de 2 à 8 cm, et, de façon de façon particulièrement préférée, de 4 cm.

11. Le corps moulé scellable selon l'une des revendications précédentes, **caractérisé en outre en ce que** la couche barrière a un effet bloquant vis-à-vis des fluides, à savoir l'eau, l'air, l'oxygène et le dioxyde de carbone.

12. Le corps moulé scellable selon l'une des revendications précédentes, **caractérisé en outre en ce que**
la couche d'étanchéité a une épaisseur moyenne comprise entre 10 et 100 µm, de préférence entre 30 et 70 µm, et, de façon particulièrement préférée, de 50 µm
la couche adhésive a une épaisseur comprise entre 1 et 10 µm, de préférence entre 2 et 5 µm, et, de façon particulièrement préférée, de 3 µm,
la couche externe de matériau non tissé a une épaisseur comprise entre 0,1 et 1 mm, de préférence entre 0,2 et 0,5 mm, et, de façon particulièrement préférée, de 0,3 mm, et
ladite au moins une couche barrière a une épaisseur comprise entre 5 et 50 µm, de préférence entre 10 et 30 µm, et, de façon particulièrement préférée, de 20 µm.

13. Le corps moulé scellable selon l'une des revendications précédentes, **caractérisé en outre en ce que** le film composite contient entre 1 et 5, de préférence 3, et, de façon particulièrement préféré, 2 couches barrières.

14. Le corps moulé scellable selon l'une des revendications précédentes, **caractérisé en outre en ce que** le film composite est thermoformable.

15. Le corps moulé scellable selon l'une des revendications précédentes, **caractérisé en outre en ce que** le film composite a une résistance à la traction de plus de 50 newtons, une résistance à la déchirure de plus de 10 newtons, une adhérence de liaison de plus de 1,7 newtons, une perméabilité de maximum 10 g/m²/24h de vapeur d'eau à 38°C et 90% d'humidité relative, une perméabilité de maximum 3 cm³/m²/24h au dioxyde de carbone à 23°C et 50% d'humidité relative et/ou une perméabilité de maximum 1,5 cm³/m²/24h à l'oxygène à 23°C et 50% d'humidité relative.

16. Le corps moulé scellable selon l'une des revendications précédentes, **caractérisé en outre en ce que** le film composite se présente sous la forme de pièces en feuille et **en ce que** le corps moulé peut être réalisé à partir de deux pièces de film composite.

17. Le corps moulé scellable selon la revendication 16, **caractérisé en outre en ce que** le film composite des pièces respectives est identique ou différent, au moins un film composite étant thermodéformable.

18. Un procédé de production d'un corps moulé scellable selon la revendication 16, dans lequel
un évidement est formé dans une pièce du film composite par thermoformage de telle sorte que la couche externe de matériau non tissé soit tournée vers l'extérieur,
la deuxième pièce de film composite selon l'une des revendications 1 à 15 est mise en contact avec la première pièce de film composite de telle sorte que les couches d'étanchéité respectives soient tournées l'une vers l'autre, et,
après avoir rempli l'évidement du corps moulé, les deux pièces du film composite sont scellées l'une à l'autre en appliquant de la chaleur aux zones de contact.

19. Utilisation d'un corps moulé scellable selon l'une quelconque des revendications 1 à 17 pour sceller des substances pharmaceutiques, en particulier des produits biologiques oraux et des vaccins oraux.
